# EUROPEAN PATENT SPECIFICATION

(11) **EP 1 937 310 B1**
(45) Date of publication and mention of the grant of the patent: **24.11.2010**
(21) Application number: 06793626.0
(22) Date of filing: 19.09.2006
(51) Int. Cl.: A61P 35/00, A61K 47/48

(54) **USE OF CONJUGATES OF DOXORUBICIN WITH LACTOSAMINATED ALBUMIN**
VERWENDUNG VON KONJUGATEN VON DOXORUBICIN MIT LACTOSAMINIERTEM ALBUMIN
UTILISATION DE CONJUGUES DE DOXORUBICINE ET D'ALBUMINE LACTOSAMINEE

(30) Priority: 20.09.2005 IT MI20051743
(43) Date of publication of application: 02.07.2008
(73) Proprietor: Universita' di Bologna Dipartimento di Patologia, 40126 Bologna (IT)
(72) Inventor: FIUME, Luigi, I-40125 Bologna (IT); DI STEFANO, Giuseppina, I-40068 San Lazzaro Di Savena (BO) (IT)
(74) Representative: Pistolesi, Roberto
(86) International application number: PCT/EP2006/066489
(87) International publication number: WO 2007/039448

(56) References cited:
- WO-A-93/25239
- WO-A2-2005/107814
- WO-A2-2006/074272
- STEFANO DI G ET AL: "Doxorubicin coupled to lactosaminated human albumin remains confined within mouse liver cells after the intracellular release from the carrier" DIGESTIVE AND LIVER DISEASE, W.B. SAUNDERS, vol. 35, no. 6, June 2003 (2003-06), pages 428-433, XP002361079 ISSN: 1590-8658
- STEFANO G D ET AL: "A novel method for coupling doxorubicin to lactosaminated human albumin by an acid sensitive hydrazone bond: synthesis, characterization and preliminary biological properties of the conjugate" EUROPEAN JOURNAL OF PHARMACEUTICAL SCIENCES, ELSEVIER, AMSTERDAM, NL, vol. 23, no. 4-5, December 2004 (2004-12), pages 393-397, XP004658708 ISSN: 0928-0987
- KRATZ F: "Drug conjugates with albumin and transferrin" EXPERT OPINION ON THERAPEUTIC PATENTS, ASHLEY PUBLICATIONS, GB, vol. 12, no. 3, 2002, pages 433-439, XP002337252 ISSN: 1354-3776
- SEOW Y-Y T ET AL: "Expression of a functional asialoglycoprotein receptor in human renal proximal tubular epithelial cells" NEPHRON, XX, XX, vol. 91, no. 3, 2002, pages 431-438, XP008066266 ISSN: 0028-2766
- TRERÈ D ET AL: "THE ASIALOGLYCOPROTEIN RECEPTOR IN HUMAN HEPATOCELLULAR CARCINOMAS: ITS EXPRESSION ON PROLIFERATING CELLS" BRITISH JOURNAL OF CANCER, LONDON, GB, vol. 81, no. 3, 1999, pages 404-408, XP008075840 ISSN: 0007-0920
- SAXENA A. ET AL: "H2, the minor subunit of the human asialoglycoprotein receptor, traffics intracellularly and forms homo-oligomers, but does not bind asialo-orosomucoid" JOURNAL OF BIOLOGICAL CHEMISTRY, vol. 277, no. 38, 2002, pages 35297-35304, XP002423100
- FIUME ET AL: "Doxorubicin coupled to lactosaminated albumin inhibits the growth of hepatocellular carcinomas induced in rats by diethylnitrosamine" JOURNAL OF HEPATOLOGY, MUNKSGAARD INTERNATIONAL PUBLISHERS, COPENHAGEN, DK, vol. 43, no. 4, October 2005 (2005-10), pages 645-652, XP005058385 ISSN: 0168-8278
- DI STEFANO G ET AL: "ENHANCED UPTAKE OF LACTOSAMINATED HUMAN ALBUMIN BY RAT HEPATOCARCINOMAS: IMPLICATIONS FOR AN IMPROVED CHEMOTHERAPY OF PRIMARY LIVER TUMORS" LIVER INTERNATIONAL, BLACKWELL MUNKSGAARD, OXFORD, GB, vol. 25, no. 4, 2005, pages 854-860, XP008075833 ISSN: 1478-3223
- DI STEFANO ET AL: "Doxorubicin coupled to lactosaminated albumin: Enhanced drug levels in rat hepatocarcinomas" DIGESTIVE AND LIVER DISEASE, W.B. SAUNDERS, vol. 38, no. 4, April 2006 (2006-04), pages 284-285, XP005347320 ISSN: 1590-8658
- DI STEFANO G ET AL: "A CONJUGATE OF DOXORUBICIN WITH LACTOSAMINATED ALBUMIN ENHANCES THE DRUG CONCENTRATIONS IN ALL THE FORMS OF RAT HEPATOCELLULAR CARCINOMAS INDEPENDENTLY OF THEIR DIFFERENTIATION GRADE" LIVER INTERNATIONAL, BLACKWELL MUNKSGAARD, OXFORD, GB, vol. 26, no. 6, 2006, pages 726-733, XP008075839 ISSN: 1478-3223
- HARRIS L ET AL: "Patterns of ligand binding to normal, regenerating, preneoplastic, and neoplastic rat hepatocytes" CANCER RESEARCH 1987 US, vol. 47, no. 15, 1987, pages 3954-3958, ISSN: 0008-5472
- ASHWELL G ET AL: "CARBOHYDRATE SPECIFIC RECEPTORS OF THE LIVER" SNELL, E. E. (ED.). ANNUAL REVIEW OF BIOCHEMISTRY, VOL. 51. XII+1055P. ANNUAL REVIEWS INC.: PALO ALTO, CALIF., USA. ILLUS SERIES : ANNUAL REVIEW OF BIOCHEMISTRY (ISSN 0066-4154), 1982, pages P531-554,
- FIUME L ET AL: "Lactosaminated human serum albumin as hepatotropic drug carrier. Rate of uptake by mouse liver" FEBS LETTERS 1982 NL LNKD- DOI:10.1016/0014-5793(82)80701-1, vol. 146, no. 1, 1982, pages 42-46, ISSN: 0014-5793

## Description

The object of the present invention is the use of a conjugate of doxorubicin with lactosaminated human albumin for the preparation of a pharmaceutical composition useful in the treatment of hepatocellular carcinomas (HCCs) which do not express the asialoglycoprotein receptor (ASGP-R). The conjugate which was previously prepared and studied only for the treatment of HCCs expressing the ASGP-R, has now been unexpectedly shown to possess the potentiality for a beneficial use also in the treatment of the HCCs which do not have the receptor. Therefore, compositions containing the conjugate could be administered for treatment of all HCCs, without the need of a preliminary tumor biopsy to demonstrate the presence or the absence of the receptor. In previous experiments we coupled doxorubicin (DOXO) to lactosaminated human albumin (L-HSA) in order to increase the anticancer activity of the drug and reduce its toxic side effects in the treatment of those hepatocellular carcinomas whose cells express the receptor for asialoglycoproteins (ASGP-R) (Di Stefano G, et al. Doxorubicin coupled to lactosaminated human albumin... Dig Liver Dis 2003; 35: 428-433; Fiume L, et al. Doxorubicin coupled to lactosaminated albumin... J Hepatol 2005; 43: 645-652). HCCs are tumors resistant to chemotherapeutic agents; DOXO is active on HCCs, but displays severe adverse reactions at the effective doses (Llovet JM. Updated treatment...J Gastroenterol 2005; 40:225-235). Toxic effects of DOXO are mainly produced on heart, bone marrow and intestine (Mazuè G, et al. Anthracyclines: a review...Int J Oncol 1995; 7: 713-26). The ASGP-R is present only on the surface of hepatocytes. It mediates uptake and lysosomal degradation of galactosyl terminating peptides (Ashwell G, et al. Carbohydrate-specific...Annu Rev Biochem 1982; 51: 531-534), which can be used as vectors for selectively delivering the drug to parenchymal liver cells (Fiume L, et al. Liver targeting of antiviral nucleoside analogs through the asialoglycoprotein receptor. J Viral Hep 1997; 4: 363-370). L-HSA is a galactosyl terminating neoglycoprotein that has been successfully used as hepatotropic drug carrier in humans (Torrani Cerenzia MR, et al. Adenine arabinoside monophosphate...Hepatology 1996; 23: 657-661; Zarski JP, et al. Efficacy and safety of L-HSA-ara-AMP...J Hepatol 2001; 34: 487-488). DOXO was coupled to L-HSA by an acid sensitive hydrazone bond that allows DOXO to be intracellularly released from the carrier in the endosomal and lysosomal compartments (Greenfield RS, et al. Evaluation in vitro of adriamycin... Cancer Res 1990; 50: 6600-6607). (The conjugation procedure is covered by a patent filed by the University of Bologna (PCT/IT2005/000257)). ASGP-R is maintained on the neoplastic cells of the majority of well differentiated (WD) human HCCs, whereas it is not expressed on the cells of the majority of the poorly differentiated (PD) HCCs (Hyodo I, et al. Distribution of asialoglycoprotein receptor...Liver 1993;13: 80-85; Trerè D, et al. The asialoglycoprotein receptor...Br J Cancer 1999; 81: 404-408; Sawamura T, et al. "Hyperasialoglycoproteinemia in patients..." Gastroenterology 1984; 87: 1217-1221). Presence or absence of the ASGP-R in human HCCs can be determined immunohistochemically using needle biopsies or fragments from surgically removed tumors. In rats with HCCs induced by diethylnitrosamine (DENA) the ability of the tumors to internalize L-HSA through the ASGP-R was found in all the 7 studied WD HCCs, in 4 out of 5 moderately differentiated (MD) tumors, whereas a low capacity to take up L-HSA was observed only in 2 out of 5 PD HCCs (Di Stefano G, et al. Enhanced uptake of lactosaminated...Liver Int 2005; 25: 854-860).

In experiments on the distribution of a radioactive L-HSA-DOXO in HCCs and organs of rats we made two unexpected and related observations:
1 - PD HCCs internalize amounts of conjugate three times higher than those of L-HSA
2 - In all the forms of HCCs, independently of their differentiation grade, the conjugate produced high concentrations of DOXO, 5-8 fold higher than those measured in intestine and heart.

### Materials and Methods

### Synthesis and characterization of L[¹⁴C]HSA-DOXO

Human albumin (HSA) was obtained by Kedrion (Lucca, Italy). It was gel-filtered on Sephacryl S-200 HR (Sigma, St Louis, MO, USA) and the monomer (> 90% of the starting material) was collected and used. HSA was labeled with [¹⁴C]-sucrose. [¹⁴C]-sucrose is a radioactive tracer which remains within the entered cells, allowing an exact determination of protein uptake in tissues (Pittman RC, et al. "Radiolabeled sucrose..." J Biol Chem. 1979; 254: 6876-6879). [¹⁴C]-sucrose (500 mCi/mmol, Amersham, Buckinghamshire, UK) was diluted to a specific activity of 3.2 x 10⁵ dpm/µg. Coupling of [¹⁴C]-sucrose to HSA was performed according to Pittman RC et al. ("Radiolabeled sucrose..." J Biol Chem. 1979; 254: 6876-6879). Since this procedure causes a protein oligomerization, labeled HSA was gel-chromatographed on a Sephacryl S200 HR column, and the monomer (70% of preparation) was collected. The molar ratio [¹⁴C]-sucrose / HSA was determined by counting the radioactivity and measuring the protein according to Lowry OH et al. ("Protein measurement..." J Biol Chem 1951; 193: 265-275)- and it was 0.2 . Alpha-lactose (Sigma) was coupled to [¹⁴C]HSA by reductive amination (Wilson G. "Effect of reductive lactosamination..." J Biol Chem 1978; 253: 2070-2072). The molar ratio lactose / [¹⁴C]HSA was determined by measuring the sugar according to Dubois M et al. ("Colorimetric method for determination..." Anal Chem 1956; 28: 350-356) and it was 26. Coupling of DOXO to L-[¹⁴C]HSA was performed using the (6-maleimidocaproyl)hydrazone derivative of the drug (DOXO-EMCH), synthesized according to Willner D, et al. ("(6-maleimidocaproyl)hydrazone derivative..." Bioconj Chem 1993; 4: 521-527). Coupling of DOXO-EMCH with L-[¹⁴C]HSA was carried out according to Di Stefano G, et al. ("A novel method for coupling..." Eur J Pharm Sci 2004; 23: 393-397), here attached for reference, but allowing the compounds to react at 20°. The molar ratio DOXO / L-[¹⁴C]HSA were calculated by measuring the protein concentration according to Lowry OH, et al. (Protein measurement...J Biol Chem 1951; 193: 265-275) and DOXO by absorbance at λ ₄₉₅ [ε₄₉₅(pH 7.4) of DOXO-EMCH = 9250 M⁻¹ cm⁻¹ ]. It was 5.8 (1 mg DOXO was contained in 24 mg conjugate; 24 mg conjugate contained 22.5 mg L-HSA).

*Induction of HCCs in rats; distribution of the conjugate and DOXO concentrations in tumors and organ.*

Male Wistar rats were used. They were obtained from Harlan Italy (Udine, Italy) and were maintained in an animal facility at the Department of Experimental Pathology, Bologna. The protocols of the experiments were approved by the Ethical Committee of the University of Bologna.

HCCs were induced by diethylnitrosamine (DENA) given in the drinking water (100 mg/l) for 8 weeks. Six to eight weeks after the last day of DENA administration, animals were i.v. injected with the following compounds: L-[¹⁴C]HSA (22.5 µg/g), L-[¹⁴C]HSA-DOXO (24 µg/g, corresponding to 22.5 µg/g of L-[¹⁴C]HSA and to 1 µg/g of DOXO) and free DOXO (1 µg/g). Compounds were injected in the dorsal vein of penis, in a volume of 10 µl/10g body weight, under isoflurane anaesthesia. For each compound four rats were used. Four hours after the injection the animals were killed under isoflurane anaesthesia. Organs were rapidly removed and frozen and neoplastic nodules were accurately dissected from the surrounding liver. A part of each tumor nodule was frozen; the other part was fixed in 10% formalin and processed for histology. Samples of frozen organs and nodules were used for radioactivity count and determination of DOXO levels. DOXO was measured according to Bots AM, et al. (Analysis of adriamycin...J Chromatogr 1983; 272: 421-427), with modifications (Di Stefano G, et al. Doxorubicin coupled to lactosaminated... Dig Liver Dis 2003; 35: 428-433).

### Results

In rats, DENA induced well, moderately and poorly differentiated forms of HCCs (WD, MD, and PD HCCs, respectively). They showed histological features super-imposable to those of human HCCs. As described by Di Stefano G et al. ("Enhanced uptake of lactosaminated..." Liver Int 2005; 25: 854-860) in WD HCCs neoplastic hepatocytes were isomorphic with eosinophilic cytoplasm; they were similar to their non-neoplastic counterpart with a trabecular pattern and intervening vascular spaces. In PD HCCs the tumor tissue showed a solid appearance; neoplastic cells were polymorphic with a basophilic cytoplasm. The MD HCCs showed a histological appearance intermediate between that of WD and that of PD HCCs. Distribution of L-[¹⁴C]HSA and of L-[¹⁴C]HSA-DOXO in HCCs, heart and intestine is reported in Table 1. In agreement with Di Stefano G et al. ("Enhanced uptake of lactosaminated..." Liver Int 2005; 25: 854-860), in four out of six examined PD HCCs, the L-[¹⁴C]HSA reached concentrations not higher than those measured in heart and intestine, organs which do not express the ASGP-R (dpm/g/SA = 9.1 ± 0.1). The conjugate L-[¹⁴C]HSA-DOXO was internalized by PD HCCs in amounts four times higher than those of L-[¹⁴C]HSA (p = 0.004, according to the Student's t-test) and entered in all the seven examined PD HCCs in quantities at least seven times higher than those taken up by heart and intestine.

An important finding was that in animals injected with the conjugate DOXO concentrations were more than eight and five times higher than those measured in heart and intestine, respectively (p = 0.006). On the contrary, in animals injected with the free drug the DOXO concentrations measured in heart and intestine were higher than those determined in HCCs (Table 2).

In conclusion, contrary to unconjugated DOXO, L-HSA-DOXO produces in all HCCs, drug concentrations higher than those in heart and intestine, target organs of toxic action of DODO, independently of the differentiation grade of the tumors and their capacity of internalizing L-HSA. As a consequence, the conjugate, formerly prepared to increase the antineoplastic efficacy and to reduce the toxicity of DOXO in the treatment of HCCs that maintain the ability of internalizing proteins exposing galactosyl residues, on the basis of the present observations, shows the potentiality for improving the chemotherapeutic index of DOXO in the treatment of all HCC forms, including the poorly differentiated ones, which display no or only poor capacity to accumulate L-HSA with respect to extra-hepatic tissues.

Therefore, the object of the present invention is the provision of L-SA-DOXO and particularly of L-HSA-DOXO for chemotherapy of HCCs that do not express the ASGP-R.

**TABLE 1**

| **Distribution of L-[¹⁴C]HSA and L-[¹⁴C]HSA-DOXO in HCCs with different differentiation grade, in heart and intestine** | | | | | |
|---|---|---|---|---|---|
| **Compound** | **dpm/g/SA^{a)}** | | | | |
| | **WD HCCs** | **MD HCCs** | **PD HCCs** | **Heart** | **Intestine** |
| L-[¹⁴C]HSA^{b)} | 178.3 ± 12.4 (6)^{d)} | 85.6 ± 4.4 (5) | 32.6 ± 14.9 (6) | 8.7 ± 0.9 | 8.9 ± 1.5 |
| L-[¹⁴C]HSA-DOXO^{c)} | 180.9 ± 27. 8 (7) | 135.5 ± 19.3 (10) | 124.8 ± 20.2 (7) | 9.0 ± 0.8 | 9.8 ± 0.8 |

Experimental details are described in Materials and Methods. Rats were killed 4h after i.v. injection of compounds. For each compound, 4 animals were used. Data are mean values ± standard error.
a) SA = Specific Activity
b) L-[¹⁴C]HSA was injected at the dose of 22.5 µg/g.
c) L-[¹⁴C]HSA-DOXO was injected at the dose of 24 µg/g (24 µg of conjugate contain 22.5 µg of L-[¹⁴C]HSA and 1 µg of DOXO).
d) Number of examined HCCs

**TABLE 2**

| **DOXO concentrations in HCCs with different differentiation grade, in heart and intestine measured after administration of L-[¹⁴C]HSA-DOXO and of free DOXO** | | | | | |
|---|---|---|---|---|---|
| **Compound** | **nmoles DOXO / g^{a)}** | | | | |
| | **WD HCCs** | **MD HCCs** | **PD HCCs** | **Heart** | **Intestine** |
| L-[¹⁴C]HSA-DOXO^{b)} | 10.2 ± 2.0 (9)^{d)} | 7.6 ± 0.9 (14) | 6.8 ± 1.1 (8) | 0.8 ± 0.1 | 1.2 ± 0.2 |
| DOXO^{c)} | 3.4 ± 0.6 (5) | 3.1 ± 0.4 (4) | 3.6 ± 0.4 (8) | 4.7 ± 0.3 | 4.2 ± 0.6 |

Experimental details are described in Materials and Methods. Data are mean values ± standard error.
a) In animals injected with the conjugate DOXO was measured as free drug. i.e. liberated from the carrier L-HSA inside the cells.
b) L-[¹⁴C]HSA-DOXO was injected at the dose of 24 µg/g (24 µg of conjugate contain 1 µg of DOXO).
c) DOXO was injected at the dose of 1 µg/g.
d) Number of examined HCCs.

## Claims

1. Use of a conjugate of doxorubicin with lactosaminated albumin for the preparation of a medicament for the treatment of the hepatocellular carcinomas which do not express the receptor for the asialoglycoproteins.

2. Use according to claim 1, **characterized in that** the administration of the conjugate does not comprise the preliminary demonstration of the presence or of the absence of the receptor for the asialoglycoproteins in the tumour cells.

3. Use according to anyone of the preceding claims, **characterized in that** said lactosaminated albumin is lactosaminated human albumin.

4. Use according to anyone of the preceding claims, **characterized in that** said medicament is administrable by parenteral route.

5. Use according to claim 4, **characterized in that** said medicament is administrable by intravenous route, by bolus or by infusion.

6. Use according to anyone of the preceding claims, **characterized in that** said medicament is an aqueous solution.

7. Use according to claim 6, **characterized in that** said aqueous solution contains excipients and/or pharmaceutically acceptable coadjuvants.

## Patentansprüche

1. Verwendung eines Doxorubicin-Konjugats mit lactosaminiertem Albumin zur Herstellung eines Arzneimittels zur Behandlung von hepatozellulären Karzinomen, die den Asialoglykoproteinrezeptor nicht exprimieren.

2. Verwendung gemäß Anspruch 1,
**dadurch gekennzeichnet, dass** die Verabreichung des Konjugats den vorläufigen Nachweis der Anwesenheit oder Abwesenheit des Asialoglykoproteinrezeptors in den Tumorzellen nicht umfasst.

3. Verwendung gemäß einem der vorhergehenden Ansprüche,
**dadurch gekennzeichnet, dass** das lactosaminierte Albumin humanes lactosaminiertes Albumin ist.

4. Verwendung gemäß einem der vorhergehenden Ansprüche,
**dadurch gekennzeichnet, dass** das Arzneimittel parenteral verabreichbar ist.

5. Verwendung gemäß Anspruch 4,
**dadurch gekennzeichnet, dass** das Arzneimittel intravenös, durch Bolusinjektion oder durch Infusion, verabreichbar ist.

6. Verwendung gemäß einem der vorhergehenden Ansprüche,
**dadurch gekennzeichnet, dass** das Arzneimittel eine wässrige Lösung ist.

7. Verwendung gemäß Anspruch 6,
**dadurch gekennzeichnet, dass** die wässrige Lösung Hilfsstoffe und/oder pharmazeutisch akzeptable Co-Adjuvantien enthält.

## Revendications

1. Utilisation d'un conjugué de la doxorubicine avec de l'albumine lactosaminée pour la préparation d'un médicament pour le traitement des carcinomes hépatocellulaires qui n'expriment pas le récepteur pour les asialoglycoprotéines.

2. Utilisation selon la revendication 1, **caractérisée en ce que** l'administration du conjugué ne comprend pas la démonstration préliminaire de la présence ou de l'absence du récepteur pour les asialoglycoprotéines dans les cellules tumorales.

3. Utilisation selon l'une quelconque des revendications précédentes, **caractérisée en ce que** ladite albumine lactosaminée est l'albumine humaine lactosaminée.

4. Utilisation selon l'une quelconque des revendications précédentes, **caractérisée en ce que** ledit médicament est administrable par voie parentérale.

5. Utilisation selon la revendication 4, **caractérisée en ce que** ledit médicament est administrable par voie intraveineuse, par bolus ou par perfusion.

6. Utilisation selon l'une quelconque des revendications précédentes, **caractérisée en ce que** ledit médicament est une solution aqueuse.

7. Utilisation selon la revendication 6, **caractérisée en ce que** ladite solution aqueuse contient des excipients et/ou des coadjuvants pharmaceutiquement acceptables.
